# EUROPEAN PATENT SPECIFICATION

(11) **EP 2 993 995 B1**
(45) Date of publication and mention of the grant of the patent: **11.04.2018**
(21) Application number: 14724674.8
(22) Date of filing: 06.05.2014
(51) Int. Cl.: A61K 8/67, A61K 8/73, A61Q 19/00, A61K 9/14, A61K 8/02, A23L 2/52, A61K 9/00, A61K 9/48, A61K 8/49, A61K 8/06, C08L 3/00, A23P 10/40, A23L 29/10, A23L 29/212, A23L 29/219, A23L 29/30, A23L 33/15

(54) **POWDEROUS VITAMIN E FORMULATION**
PULVERFÖRMIGE VITAMIN-E-FORMULIERUNG
FORMULATION DE VITAMINE E PULVÉRULENTE

(30) Priority: 06.05.2013 EP 13166666
(43) Date of publication of application: 16.03.2016
(73) Proprietor: DSM IP Assets B.V., 6411 TE Heerlen (NL)
(72) Inventor: VOELKER, Karl Manfred, CH-4002 Basel (CH); BADOLATO-BÖNISCH, Gabriela, CH-4002 Basel (CH); LINDEMANN, Thomas, CH-4002 Basel (CH); HITZFELD, Andrea, CH-4002 Basel (CH); KIRCHEN, Stefanie, CH-4002 Basel (CH)
(74) Representative: Kurt, Manfred
(86) International application number: PCT/EP2014/059207
(87) International publication number: WO 2014/180827

(56) References cited:
- EP-A1- 0 966 889
- EP-A2- 1 066 761
- WO-A1-2007/090614
- WO-A1-2011/039336
- WO-A1-2011/162802
- WO-A2-2009/000404
- WO-A2-2009/010305
- WO-A2-2010/025335
- US-A1- 2003 104 078
- US-A1- 2011 280 852

## Description

The present invention relates a powderous formulation comprising vitamin E, which can be produced easily and which can be used in many fields of application, but mainly in beverages.

The term "vitamin E" in the context of the present patent application covers vitamin E and its esters (such as and especially vitamin E acetate).

The vitamin E and its esters can be from a natural source or it can be synthesised. Due to the nature of either the isolation process or the process of production, it is possible that traces of side products are present.

The eight forms of vitamin E are divided into two groups; four are tocopherols and four are tocotrienols. They are identified by prefixes alpha- (α-), beta- (β-), gamma- (γ-), and delta-(δ-). Natural tocopherols occur in the RRR-configuration only. The synthetic form contains eight different stereoisomers and is called dl-α-tocopherol.

Vitamin E in the context of the present invention is preferably vitamin E acetate and dl-α-tocopherol acetate.

Many formulations (liquid and solid) comprising vitamin E are known from the prior art.

EP966889A1 discloses droplets of a fat soluble vitamin wherein the droplets average about 70 to about 200 nanometers in diameter, and which are dispersed in a modified polysaccharide matrix, beverages and tablets containing the composition, and methods of making the composition.

Due to the importance of vitamin E there is always a need for improved formulations (in regard to the formulations, as well as in regard of their production and in regard to their use). Furthermore, there are usually issues during the production (spray drying process) in regard to the stickiness of the formulation.

Surprisingly it has been found that a powderous composition comprising vitamin E and a specific maltodextrin and a modified polysaccharide and a polyoxyethylene sorbitan mono fatty acid ester has improved properties.
One surprising advantage of the powderous composition according to the present invention is that the composition is not sticky (even during the drying process, which is usually done by spray-drying). The composition does not stick to the wall of the drying apparatus. Therefore the loss of material during the production is decreased as well as the time for cleaning is shortened.

Furthermore when these formulations are used in liquid formulations (such as beverages), they result in non-turbid (transparent, non-opaque) liquid formulations (such as beverages).

Therefore the present invention relates to a powderous composition (I) comprising
(i) 5 to 25 weight-% (wt-%), based on the total weight of the powderous composition, of dl-α-tocopherol acetate, and
(ii) 20 - 50 wt-%, based on the total weight of the powderous composition, of at least one maltodextrin having a DE of < 20, and
(iii) 20 - 50 wt-%, based on the total weight of the powderous composition, of at least one modified polysaccharide, and
(iv) at least 5 wt-%, based on the total weight of the powderous composition, of at least one polyoxyethylene sorbitan monofatty acid ester.

It is clear that the percentages always add up to 100.

The composition according to the present invention is a dry powder. Nevertheless it can also comprise some water, which originates from the emulsion. Usually and preferably, the water content is less than 5 wt-%, based on the total weight of the powderous composition. Usually less than 4wt-%,.

The composition according to the present invention comprises a maltodextrin having a DE of < 20.

Dextrose equivalent (DE) is a measure of the amount of reducing sugars present in a sugar product, relative to glucose, expressed as a percentage on a dry basis. For example, a maltodextrin with a DE of 10 would have 10% of the reducing power of dextrose (which has a DE of 100). Maltose, a disaccharide made of two glucose (dextrose) molecules has a DE of 52, correcting for the water loss in molecular weight when the two molecules are combined (180/342). Sucrose actually has a DE of 0 even though it is a disaccharide, because both reducing groups of the monosaccharides that make it are connected, so there are no remaining reducing groups. For solutions made from starch, it is an estimate of the percentage reducing sugars present in the total starch product.

In all glucose polymers, from the native starch to glucose syrup, the molecular chain begins with a reducing sugar, containing a free aldehyde. As the starch is hydrolysed, the molecules become shorter and more reducing sugars are present. Because different reducing sugars (e.g. fructose and glucose) have different sweetness, it is incorrect to assume that there is any direct relationship between DE and sweetness.
The DE describes the degree of conversion of starch to dextrose:
starch is close to DE = 0,
glucose/dextrose is DE = 100 (percent),

The standard method of determining DE is the Lane-Eynon titration, based on the reduction of copper(II) sulfate in an alkaline tartrate solution, an application of Fehling's test.

Preferred is a maltodextrin having a DE< 18.

Therefore the present invention relates to a composition (III), which is composition (I), wherein the maltodextrin has a DE< 18.

The maltodextrin used in the composition according to the present invention can be from different sources.

Preferably, the maltodextrin is from a corn source or pea source, more preferably from a pea source.

Therefore the present invention relates to a composition (IV), which is composition (I) or (III), wherein the maltodextrin is from a corn source or pea source.

Therefore the present invention relates to a composition (V), which is composition (I), (III) or (IV), wherein the maltodextrin is from a pea source.

When using these maltodextrin types - especially the one sourced from pea (in contrast to other common sources, like potato, wheat, etc)-, the production process is improved significantly.

The composition according to present invention comprises at least one modified polysaccharide.

Preferably the modified polysaccharide is modified starch.

Therefore the present invention relates to a composition (VI), which is composition (I), (III), (IV) or (V), wherein the modified polysaccharide is modified starch.

Preferably the modified polysaccharide is of formula (I) 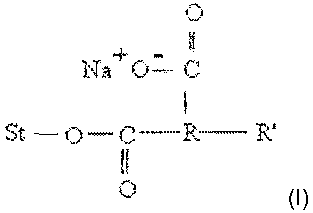 wherein
St is a starch,
R is an alkylene group and R' is a hydrophobic group.

Therefore the present invention relates to a composition (VII), which is composition (I), (III), (IV), (V) or (VI), wherein the modified polysaccharide is of formula (I) 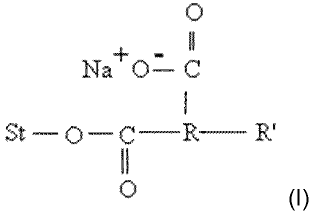 wherein
St is a starch,
R is an alkylene group and R' is a hydrophobic group.

Preferably the modified polysaccharide is starch sodium octenyl succinate.
Therefore the present invention relates to a composition (VIII), which is composition (I), (III), (IV), (V), (VI) or (VII), wherein the modified polysaccharide is starch sodium octenyl succinate.

The composition according to the present invention comprises at least one polyoxyethylene sorbitan monofatty acid ester.

Preferably the polyoxyethylene sorbitan monofatty acid ester is chosen from the group consisting of polyoxyethylene(20) sorbitan monolaurate, poly-oxyethylene(20) sorbitan-monopalmitate, polyoxyethylene(20) sorbitan monostearate and polyoxyethylene(20) sorbitan monooleate, more preferably polyoxyethylen(20)- sorbitan- monooleate.

Therefore the present invention relates to a composition (IX), which is composition (I), (III), (IV), (V), (VI), (VII) or (VIII), wherein the polyoxyethylene sorbitan monofatty acid ester is chosen from the group consisting of polyoxyethylene(20) sorbitan monolaurate, poly-oxyethylene(20) sorbitan-monopalmitate, polyoxyethylene(20) sorbitan monostearate and polyoxyethylene(20) sorbitan monooleate.

Therefore the present invention relates to a composition (X), which is composition (I), (III), (IV), (V), (VI), (VII), (VIII) or (IX), wherein the polyoxyethylene sorbitan monofatty acid ester is polyoxyethylen(20)- sorbitan- monooleate.

Preferably, the powderous composition according to present invention has an average inner particle size D [0,5] (inner phase) of less than 150 nm, more preferably less than 120 nm, most preferably 70nm - 110nm.

All the sizes of the inner phase D [0,5] in the context of the present patent application were determined by using a Mastersizer 2000. The particle size of the inner phase was determined after redispersing the powderous composition in water.

Therefore the present invention relates to a composition (XI), which is composition (I), (III), (IV), (V), (VI), (VII), (VIII), (IX) or (X), wherein the powderous composition has an average inner particle size D [0,5] (inner phase) of less than 150 nm.

Therefore the present invention relates to a composition (XII), which is composition (I), (III), (IV), (V), (VI), (VII), (VIII), (IX), (X) or (XI), wherein the powderous composition has an average inner particle size D [0,5] (inner phase) of less than 120 nm.

Therefore the present invention relates to a composition (XIII), which is composition (I), (III), (IV), (V), (VI), (VII), (VIII), (IX), (X), (XI) or (XII), wherein the powderous composition has an average inner particle size D [0,5] (inner phase) of 70nm - 110nm.

Preferably the powderous composition according to the present invention comprises 10 to 20 wt-%, based on the total weight of the powderous composition, of dl-α-tocopherol acetate.

Therefore the present invention relates to a composition (XV), which is composition (I), (III), (IV), (V), (VI), (VII), (VIII), (IX), (X), (XI), (XII) or (XIII), wherein the powderous composition comprises 10 to 20 wt-%, based on the total weight of the powderous composition, of dl-α-tocopherol acetate.

Preferably the powderous composition according to the present invention comprises 25 - 45 wt-%, based on the total weight of the powderous composition, of at least one maltodextrin having a DE of < 20.

Therefore the present invention relates to a composition (XVI), which is composition (I), (III), (IV), (V), (VI), (VII), (VIII), (IX), (X), (XI), (XII), (XIII) or (XV), wherein the powderous composition comprises 25 - 45 wt-%, based on the total weight of the powderous composition, of at least one maltodextrin having a DE of < 20.

Preferably, the powderous composition according to the present invention comprises 25 - 45 wt-%, based on the total weight of the powderous composition, of at least one modified polysaccharide.

Therefore the present invention relates to a composition (XVII), which is composition (I), (III), (IV), (V), (VI), (VII), (VIII), (IX), (X), (XI), (XII), (XIII), (XV) or (XVI), wherein the powderous composition comprises 25 - 45 wt-%, based on the total weight of the powderous composition, of at least one modified polysaccharide.

Preferably, the powderous composition according to the present invention comprises 5 - 20 wt-%, based on the total weight of the powderous composition, of at least one polyoxyethylene sorbitan monofatty acid ester.

Therefore the present invention relates to a composition (XVIII), which is composition (I), (III), (IV), (V), (VI), (VII), (VIII), (IX), (X), (XI), (XII), (XIII), (XV), (XVI) or (XVII), wherein the powderous composition comprises 5 - 20 wt-%, based on the total weight of the powderous composition, of at least one polyoxyethylene sorbitan monofatty acid ester.

Preferably the powderous composition according to the present invention is a spray dried composition.
Therefore the present invention relates to a composition (XIX), which is composition (I), (III), (IV), (V), (VI), (VII), (VIII), (IX), (X), (XI), (XII), (XIII), (XV), (XVI), (XVII) or (XVIII), wherein the powderous composition is a spray dried composition.

The powderous composition according to the present invention can be produced by using technologies known to a person skilled in the art.

In first step an emulsion comprising all ingredients ((i) - (iv)) and water is produced, which is then dried (usually and preferably by spray drying). The water content of the powderous composition depends on the conditions of the applies drying process.

One advantage of the powderous composition according to the present invention is that during the spray-drying procedure, the powderous composition is not sticky and therefore the powderous composition does not stick to the wall of the spray drying tower and therefor the loss of the powderous composition during the drying process is very low and the effort to clean the drying apparatus is lowered.

Therefore the present invention relates to process for production of composition (I), (III), (IV), (V), (VI), (VII), (VIII), (IX), (X), (XI), (XII), (XIII), (XV), (XVI), (XVII), (XVIII) or (XIX), characterized in that in a first step an emulsion comprising all ingredients (i) - (iv) and water is produced, which is then in a second step dried to form a powderous composition.

Therefore the present invention also relates to process for production of composition (I), (III), (IV), (V), (VI), (VII), (VIII), (IX), (X), (XI), (XII), (XIII), (XV), (XVI), (XVII), (XVIII) or (XIX), characterized in that in a first step an emulsion comprising all ingredients (i) - (iv) and water is produced, which is then in a second step spray-dried to form a powderous composition.

The powderous compositions (I), (III), (IV), (V), (VI), (VII), (VIII), (IX), (X), (XI), (XII), (XIII), (XV), (XVI), (XVII), (XVIII) and (XIX) according to present invention are used in food, feed and/or personal care formulations.

Preferably the powderous compositions (I), (III), (IV), (V), (VI), (VII), (VIII), (IX), (X), (XI), (XII), (XIII), (XV), (XVI), (XVII), (XVIII) and (XIX) are used in a liquid formulation, preferably in a beverage.

One advantage of the powderous composition is, when added to a liquid formulation the resulting formulation is not turbid. This means that the powderous compositions according to the present invention can be used for the production of clear beverages.
Furthermore, these clear beverages stay clear during storage (stable clear beverage).

The turbidity of the liquid and transparent formulation comprising at least one of the above described powderous composition is usually less than 10 NTU (for 30ppm vitamin E concentration).

The measurement of the turbidity is done by using standard methods (EN27027; ISO7027). All the measurements for this patent application are done by using a turbiditymeter Hach 2100N IS® from Hach Company, Loveland, Colorado (USA). The turbidity is given in nephe-lometric turbidity units (NTU). The measurement angle was 90° +-2,5° and the measurement wavelength was: 860nm +-10nm LED. The measurements were done at room temperature.

Furthermore the present invention relates to food, feed and personal care formulations comprising at least one powderous composition (I), (III), (IV), (V), (VI), (VII), (VIII), (IX), (X), (XI), (XII), (XIII), (XV), (XVI), (XVII), (XVIII) and/or (XIX).
These formulations can be in any form. Solid, liquid or gel-like.

Preferred are liquid food, feed and personal care formulation comprising at least one powderous composition (I), (III), (IV), (V), (VI), (VII), (VIII), (IX), (X), (XI), (XII), (XIII), (XV), (XVI), (XVII), (XVIII) and/or (XIX).
More preferred are beverages comprising at least one powderous composition (I), (III), (IV), (V), (VI), (VII), (VIII), (IX), (X), (XI), (XII), (XIII), (XV), (XVI), (XVII), (XVIII) and/or (XIX).

Therefore a further embodiment of the present invention relates to food, feed and personal care formulations comprising at least one powderous composition (I), (III), (IV), (V), (VI), (VII), (VIII), (IX), (X), (XI), (XII), (XIII), (XV), (XVI), (XVII), (XVIII) and/or (XIX).

Therefore a further embodiment of the present invention relates to liquid food, feed and personal care formulations comprising at least one powderous composition (I), (III), (IV), (V), (VI), (VII), (VIII), (IX), (X), (XI), (XII), (XIII), (XV), (XVI), (XVII), (XVIII) and/or (XIX).

Therefore a further embodiment of the present invention relates to beverages comprising at least one powderous composition (I), (III), (IV), (V), (VI), (VII), (VIII), (IX), (X), (XI), (XII), (XIII), (XV), (XVI), (XVII), (XVIII) and/or (XIX).

The concentration of the powderous composition in the food, feed and/or personal care formulations depends on the kind of these formulations.

The invention is illustrated by the following Examples. All temperatures are given in °C and all parts and percentages are related to the weight.

### Example 1:

140 g Capsul HS and 105 g Kleptose Linecaps 17 were put into a 1.5 l reaction vessel and were dispersed in 150 g deionised water at room temperature. The temperature was increased to 85 °C under stirring with a micer disk (800 rpm, d=6 cm). The solution was kept at 85 °C for 60 min. 56 g dl-alpha-tocopherolacetate was preheated to 65 °C and added within 5-10 min to the mixture of Capsul HS, Kleptose Linecaps 17 and water under homogenizing with the micer disk (8'500 rpm). Simultaneously 51.5 g water was added to the emulsion within 5-10 min. This emulsion was homogenized for another 30 min. at 85 °C with the micer disk (8'500 rpm). Then 36.8 g Lamesorb SMO 20 was preheated to 65 °C and added to the emulsion within 5 min under homogenizing (micer disk (8'500 rpm). The emulsion temperature was kept at 85 °C. Then the emulsion was homogenized for another 30 min. at 85 °C (micer disk, 8'500 rpm).
The emulsion was kept at 85 °C for another 30 min. under stirring (micer disk, 250 rpm). The median particle size was < 100 nm.

Afterwards this emulsion was dried in a spray drying process (emulsion temperature: 85 - 65 °C). The temperature at the inlet of the spray drying tower was between 179 - 184 °C and the temperature at the outlet of the spray drying tower was bwetween 80 - 96°C.
A powderous composition was obtained with a residual moisture content of 3.1%. The size of the inner phase D[0,5] was 93 nm.
The following table shows the amounts of the ingredients of the composition (the values are recalculated for a water-free composition).

**Table 1:**

| **Ingredient** | **Amount [%]** |
|---|---|
| Capsul HS (National Starch) | 41.4 |
| Kleptose Linecaps 17 (Roquette) | 31.1 |
| dl-α-tocopherolacetate (DSM) | 16.6 |
| Lamesorb SMO 20 (Cognis) | 10.9 |

### Application Examples

The powderous composition according to Example was used in the following application examples.
For the application examples the following basic composition of a flavoured water was used.

**Table 2:**

| **Ingredients** | **Amount [g]** |
|---|---|
| Deion water | 40.3 |
| Potassium sorbate | 0.2 |
| Crystaline sugar | 7.2 |
| Citric acid (50% aq. Solution) | 2.0 |
| Lemon flavour 78839-76 | 0.2 |
| Ginger ale flavour 60131-76 | 0.1 |

The powderous composition according to Example 1 was added to this basic composition. (the powderous composition could be added as such or in the form of a diluted stock solution).
For all the following application examples The powderous composition of Example 1 was diluted with deionized water to a stock solution having a vitamin E content of 1000ppm. This stock solution was then used to produce the samples of the application examples. All the samples were filled into glass bottles (200 ml) and the bottles were sealed with a crown cap.

Samples having a vitamin E content of
5ppm, 10ppm, 15ppm, 20ppm, 25ppm and 30ppm have been prepared.

Some of the samples have been pasteurized.
Pasteurization of the liquid formulation was conducted in a bath of water. The bottles (glass bottles/200 ml) were placed in an 85 °C water bath. After reaching 80 °C, samples remained in the water bath for 1 additional minute. A reference bottle containing water and a thermometer was used for the control of the temperature during pasteurization. After pasteurization bottles are quickly cooled (using cold water) to room temperature.

The NTU of the formulations was measured before and after pasteurization.

**Table 3:**

| **Vitamin E content** | **NTU (non-past.)** | **NTU (past.)** |
|---|---|---|
| 5ppm | 2.36 | 1.70 |
| 10ppm | 4.08 | 2.41 |
| 15ppm | 5.38 | 3.11 |
| 20ppm | 6.77 | 3.97 |
| 25ppm | 7.82 | 5.25 |
| 30ppm | 9.55 | 6.39 |

Even the formulation comprising a high amount of vitamin E has a excellent NTU value. The formulation is clear (not-turbid).

Furthermore storage tests have been carried out with vitamin E fortified flavor water composition. The basic flavoured water was the same as for the first series of application examples. The concentration of vitamin E was 26.8 mg/l.
The samples were also filled in glass bottles (200ml) and sealed with a crown cap. The samples were stored at room temperature for 2 weeks, 1 month, 2 months and 3 months. The turbidity was measured at these times.

The NTU of the pasteurized formulation as well as of the non-pasteurized formulation was not deteriorating after a storage time of 90 days.
For the non-pasteurized the NTU was around 7 (at the begining as well as after 90 days) for the pasteurized formulation the NTU was 4 (at the start as well as after 90 days).

## Claims

1. Powderous composition comprising
(i) 5 to 25 wt-%, based on the total weight of the powderous composition, of dl α tocopherol acetate, and
(ii) 20 - 50 wt-%, based on the total weight of the powderous composition, of at least one maltodextrin having a DE of < 20, and
(iii) 20 - 50 wt-%, based on the total weight of the powderous composition, of at least one modified polysaccharide, and
(iv) at least 5 wt-%, based on the total weight of the powderous composition, of at least one polyoxyethylene sorbitan monofatty acid ester.

2. Powderous composition according to claim 1, wherein the maltodextrin has a DE< 18.

3. Powderous composition according to claim 1, wherein the maltodextrin is from a corn source or pea source, preferably from a pea source.

4. Powderous composition according to any of the preceding claims, wherein the modified polysaccharide is modified starch.

5. Powderous composition according to any of the preceding claims, wherein the modified polysaccharide is of formula (I) 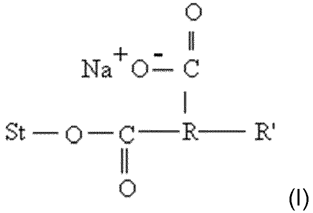 wherein St is a starch, R is an alkylene group and R' is a hydrophobic group.

6. Powderous composition according to any of the preceding claims, wherein the modified polysaccharide is starch sodium octenyl succinate.

7. Powderous composition according to any of the preceding claims, wherein the polyoxyethylene sorbitan monofatty acid ester chosen from the group consisting of polyoxyethylene(20) sorbitan monolaurate, poly-oxyethylene(20) sorbitan-monopalmitate, polyoxyethylene(20) sorbitan monostearate and polyoxyethylene(20) sorbitan monooleate, preferably polyoxyethylen(20)- sorbitan- monooleate.

8. Powderous composition according to any of the preceding claims, wherein the average inner particle size (inner phase) is less than 150 nm, preferablv less than 120 nm, more preferably 70nm - 110nm.

9. Powderous composition according to any of the preceding claims, comprising 10 - 20 wt-%, based on the total weight of the powderous composition, of dl α tocopherol acetate.

10. Powderous composition according to any of the preceding claims, comprising 25 - 45 wt-%, based on the total weight of the powderous composition, of at least one maltodextrin having a DE of < 20.

11. Powderous composition according to any of the preceding claims, comprising 25 - 45 wt-%, based on the total weight of the powderous composition, of at least one modified polysaccharide.

12. Powderous composition according to any of the preceding claims, comprising 5 - 20 wt-%, based on the total weight of the powderous composition, of at least one polyoxyethylene sorbitan monofatty acid ester.

13. Powderous composition according to any of the preceding claims, wherein the powderous composition is spray dried.

14. Process of production of any of the powderous compositions according to anyone of the preceding claims, using spray drying technology.

15. Use of a powderous composition according to anyone of the claims 1 - 12 in a liquid formulation, preferably a beverage.

16. A liquid formulation comprising at least one of the powderous composition according to anyone of claim 1- 13.

## Patentansprüche

1. Pulverförmige Zusammensetzung, umfassend:
(i) 5 bis 25 Gew.-% dl-α-Tocopherolacetat in Bezug auf das Gesamtgewicht der pulverförmigen Zusammensetzung, und
(ii) 20 - 50 Gew.-% mindestens eines Maltodextrins mit einem DE von < 20 in Bezug auf das Gesamtgewicht der pulverförmigen Zusammensetzung, und
(iii) 20 - 50 Gew.-% mindestens eines modifizierten Polysaccharids in Bezug auf das Gesamtgewicht der pulverförmigen Zusammensetzung, und
(iv) mindestens 5 Gew.-% mindestens eines Polyoxyethylensorbitanmonofettsäureesters in Bezug auf das Gesamtgewicht der pulverförmigen Zusammensetzung.

2. Pulverförmige Zusammensetzung nach Anspruch 1, wobei das Maltodextrin ein DE von < 18 aufweist.

3. Pulverförmige Zusammensetzung nach Anspruch 1, wobei das Maltodextrin von einem Maisausgangsmaterial oder einem Erbsenausgangsmaterial, vorzugsweise von einem Erbsenausgangsmaterial, stammt.

4. Pulverförmige Zusammensetzung nach einem der vorhergehenden Ansprüche, wobei es sich bei dem modifizierten Polysaccharid um modifizierte Stärke handelt.

5. Pulverförmige Zusammensetzung nach einem der vorhergehenden Ansprüche, wobei das modifizierte Polysaccharid die Formel (I) aufweist, 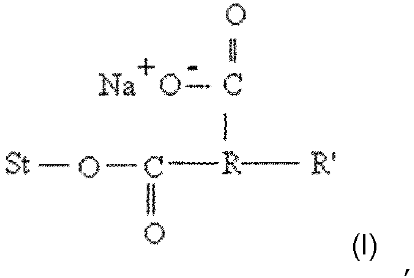 wobei St eine Stärke bedeutet, R eine Alkylengruppe bedeutet und R' eine hydrophobe Gruppe bedeutet.

6. Pulverförmige Zusammensetzung nach einem der vorhergehenden Ansprüche, wobei es sich bei dem modifizierten Polysaccharid um Natriumoctenylsuccinat handelt.

7. Pulverförmige Zusammensetzung nach einem der vorhergehenden Ansprüche, wobei der Polyoxyethylensorbitanmonofettsäureester ausgewählt aus der Gruppe bestehend aus Polyoxyethylen(20)sorbitanmonolaurat, Polyoxyethylen(20)sorbitanmonopalmitat, Polyoxyethylen(20)sorbitanmonostearat und Polyoxyethylen(20)sorbitanmonooleat, vorzugsweise Polyoxyethylen(20)sorbitanmonooleat.

8. Pulverförmige Zusammensetzung nach einem der vorhergehenden Ansprüche, wobei die durchschnittliche innere Teilchengröße (innere Phase) weniger als 150 nm, vorzugsweise weniger als 120 nm, stärker bevorzugt 70 nm - 110 nm, beträgt.

9. Pulverförmige Zusammensetzung nach einem der vorhergehenden Ansprüche, umfassend 10 - 20 Gew.-% dl-α-Tocopherolacetat in Bezug auf das Gesamtgewicht der pulverförmigen Zusammensetzung.

10. Pulverförmige Zusammensetzung nach einem der vorhergehenden Ansprüche, umfassend 25 - 45 Gew.-% an mindestens einem Maltodextrin mit einem DE von < 20 in Bezug auf das Gesamtgewicht der pulverförmigen Zusammensetzung.

11. Pulverförmige Zusammensetzung nach einem der vorhergehenden Ansprüche, umfassend 25 - 45 Gew.-% an mindestens einem modifizierten Polysaccharid in Bezug auf das Gesamtgewicht der pulverförmigen Zusammensetzung.

12. Pulverförmige Zusammensetzung nach einem der vorhergehenden Ansprüche, umfassend 5 - 20 Gew.-% an mindestens einem Polyoxyethylensorbitanmonofettsäureester in Bezug auf das Gesamtgewicht der pulverförmigen Zusammensetzung.

13. Pulverförmige Zusammensetzung nach einem der vorhergehenden Ansprüche, wobei die pulverförmige Zusammensetzung sprühgetrocknet ist.

14. Verfahren zur Herstellung von einer der pulverförmigen Zusammensetzungen nach einem der vorhergehenden Ansprüche unter Verwendung der Sprühtrocknungstechnik.

15. Verwendung einer pulverförmigen Zusammensetzung nach einem der Ansprüche 1 -12 in einer flüssigen Formulierung, vorzugsweise einem Getränk.

16. Flüssige Formulierung, umfassend mindestens eine der pulverförmigen Zusammensetzung nach einem der Ansprüche 1 - 13.

## Revendications

1. Composition pulvérulente, comprenant
(i) de 5 à 25% en poids, sur la base du poids total de la composition pulvérulente, d'acétate de dl-α-tocophérol, et
(ii) 20-50% en poids, sur la base du poids total de la composition pulvérulente, d'au moins une maltodextrine ayant un DE < 20, et
(iii) 20-50% en poids, sur la base du poids total de la composition pulvérulente, d'au moins un polysaccharide modifié, et
(iv) au moins 5% en poids, sur la base du poids total de la composition pulvérulente, d'au moins un ester de monoacide gras et de sorbitane polyoxyéthyléné.

2. Composition pulvérulente selon la revendication 1, dans laquelle la maltodextrine possède un DE < 18.

3. Composition pulvérulente selon la revendication 1, dans laquelle la maltodextrine est issue d'une source de maïs ou d'une source de pois, préférablement d'une source de pois.

4. Composition pulvérulente selon l'une quelconque des revendications précédentes, dans laquelle le polysaccharide modifié est un amidon modifié.

5. Composition pulvérulente selon l'une quelconque des revendications précédentes, dans laquelle le polysaccharide modifié répond à la formule (I) 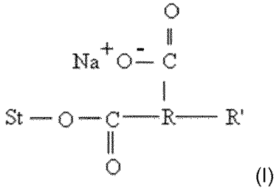 où St est un amidon, R est un groupement alkylène et R' est un groupement hydrophobe.

6. Composition pulvérulente selon l'une quelconque des revendications précédentes, dans laquelle le polysaccharide modifié est l'octénylsuccinate d'amidon sodique.

7. Composition pulvérulente selon l'une quelconque des revendications précédentes, dans laquelle l'ester de monoacide gras et de sorbitane polyoxyéthyléné choisi dans le groupe constitué par le monolaurate de sorbitane et de polyoxyéthylène (20), le monopalmitate de sorbitane et de polyoxyéthylène (20), le monostéarate de sorbitane et de polyoxyéthylène (20) et le monooléate de sorbitane et de polyoxyéthylène (20), préférablement le monooléate de sorbitane et de polyoxyéthylène (20).

8. Composition pulvérulente selon l'une quelconque des revendications précédentes, dans laquelle la taille de particules interne moyenne (phase interne) est inférieure à 150 nm, préférablement inférieure à 120 nm, plus préférablement de 70 nm-110 nm.

9. Composition pulvérulente selon l'une quelconque des revendications précédentes, comprenant 10-20% en poids, sur la base du poids total de la composition pulvérulente, d'acétate de dl-α-tocophérol.

10. Composition pulvérulente selon l'une quelconque des revendications précédentes, comprenant 25-45% en poids, sur la base du poids total de la composition pulvérulente, d'au moins une maltodextrine ayant un DE < 20.

11. Composition pulvérulente selon l'une quelconque des revendications précédentes, comprenant 25-45% en poids, sur la base du poids total de la composition pulvérulente, d'au moins un polysaccharide modifié.

12. Composition pulvérulente selon l'une quelconque des revendications précédentes, comprenant 5-20% en poids, sur la base du poids total de la composition pulvérulente, d'au moins un ester de monoacide gras et de sorbitane polyoxyéthyléné.

13. Composition pulvérulente selon l'une quelconque des revendications précédentes, où la composition pulvérulente est séchée par pulvérisation.

14. Procédé de production de l'une quelconque parmi les compositions pulvérulentes selon l'une quelconque des revendications précédentes, à l'aide d'une technologie de séchage par pulvérisation.

15. Utilisation d'une composition pulvérulente selon l'une quelconque des revendications 1-12, dans une formulation liquide, préférablement une boisson.

16. Formulation liquide comprenant au moins l'une de la composition pulvérulente selon l'une quelconque des revendications 1-13.
